# EUROPÄISCHE PATENTSCHRIFT

(11) **EP 1 166 752 B1**
(45) Veröffentlichungstag und Bekanntmachung des Hinweises auf die Patenterteilung: **07.04.2004**
(21) Anmeldenummer: 01114735.2
(22) Anmeldetag: 22.06.2001
(51) Int. Cl.: A61K 7/13

(54) **Haarfärbemittel**
Hair dyeing composition
Composition de teinture pour cheveux

(30) Priorität: 23.06.2000 DE 10031014
(43) Veröffentlichungstag der Anmeldung: 02.01.2002
(60) Teilanmeldung: 03012471.3
(73) Patentinhaber: KPSS-Kao Professional Salon Services GmbH, 64297 Darmstadt (DE)
(72) Erfinder: Grit, Mustafa, Dr., 64579 Gernsheim (DE)

(56) Entgegenhaltungen:
- EP-A- 0 819 422
- EP-A- 0 970 684

## Beschreibung

Die vorliegende Erfindung betrifft ein Haarfärbemittel mit verbesserten Gebrauchseigenschaften, insbesondere verbesserter Lichtbeständigkeit und, damit verbunden, einer verbesserten Färbeintensität und Farbstabilität.

Es ist bereits seit längerem bekannt, Haarfärbemitteln, insbesondere auf Basis direktziehender Haarfarbstoffe, UV-Absorber, beispielsweise mit anionischen Gruppen, zuzusetzen, um die Haarfärbung gegen schädlichen Lichteinfluß zu schützen.
Je stärker diese Verbindungen auf das Haar aufziehen, desto besser logischerweise der erzielte Lichtschutz der Färbung.
Dieser ist jedoch oftmals nicht optimal, da beispielsweise durch verschiedene Bestandteile von Haarfärbemittteln, insbesondere die eingesetzten Farbstoffe, das Aufziehen dieser UV-Absorber ver- oder zumindest behindert wird.

Dies gilt natürlich insbesondere für Zusammensetzungen, die beide Substanzgruppen, nämlich sowohl UV-Absorber als auch direktziehende Haarfarbstoffe enthalten, was insofern für die Praxis von Bedeutung ist, als durch die gleichzeitige Verwendung von Direktziehem und UV-Absorbern die vorzeitige Ausbleichung der erzielten Haarfärbung durch Lichteinfluß verhindert werden kann.

Die Erfindung geht daher von der Aufgabenstellung aus, ein Haarfärbemittel, das anionische UV-Absorber und direktziehende Haarfarbstoffe enthält, zu schaffen, das die erwähnten Nachteile nicht aufweist, sondern einen stabilen Lichtschutz und eine dauerhafte Haarfärbung gewährleistet.

Diese Aufgabe wird dadurch gelöst, daß in einem Haarfärbemittel auf wäßriger Basis, das mindestens einem kationischen direktziehenden Farbstoff mit der nachfolgenden Formel I 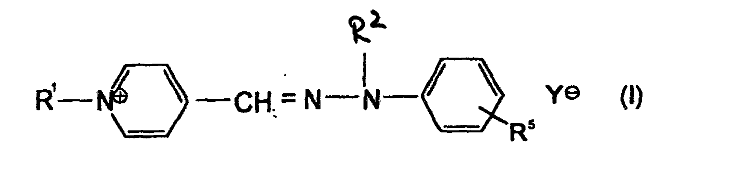 worin R¹ und R², für Wasserstoff, eine CH₃- oder C₂H₅-Gruppe stehen, und R⁵ Wasserstoff, -OCH₃ oder -OC₂H₅ bedeutet, enthält, mit mindestens einem vorzugsweise wasserlöslichen anionischen UV-Absorber kombiniert wird.

Die bevorzugte Menge an kationischem Farbstoff nach Formel I liegt bei etwa 0,001 bis etwa 5, insbesondere etwa 0,01 bis 2,5, vor allem etwa 0,1 bis etwa 1 Gew.-%, berechnet auf das Haarfärbemittel.

Es handelt sich dabei vorzugsweise um Farbstoffe der allgemeinen Formel (I), worin R¹ und R² eine Methyl- und R⁵ eine Methoxygruppe oder Wasserstoff bedeuten.

Das Anion Y⁻ ist vorzugsweise CH₃SO₄⁻, C₂H₅SO₄⁻ oder Cl⁻.

Besonders geeignete wasserlösliche UV-Absorber mit anionischen Gruppen sind beispielsweise 5-Benzoyl-4-hydroxy-2-methoxybenzolsulfonsäure (Benzophenone-4), dessen Natriumsalz (Benzophenone-5) und 2,2'-Dihydroxy-4,4'-dimethoxy-3,3'-disulfobenzophenon bzw. dessen Dinatriumsalz (Benzophenone-9) sowie Phenylbenzimidazolsulfonsäure (Eusolex® 232); jedoch können auch andere anionische UV-Absorber eingesetzt werden.

Der Anteil der direktziehenden kationischen Haarfarbstoffe in den erfindungsgemäßen Zusammensetzungen ist variabel und liegt zwischen etwa 0,001 bis etwa 5, vorzugsweise 0,01 bis 2,5, insbesondere 0,1 bis 1 Gew.-% des Mittels, falls es als Lösung, Dispersion, Emulsion, Gel oder Aerosolpräparat zur direkten Anwendung, d.h., ohne vorherige Verdünnung vorliegt.
Bei Konzentraten, die vor der Anwendung verdünnt werden, ist der Anteil natürlich entsprechend höher.

Zusätzlich zu dem genannten kationischen Farbstoff der allgemeinen Formel (I) können auch bekannte weitere synthetische Farbstoffe und pflanzliche Farbstoffe Verwendung finden, beispielsweise Henna (rot oder schwarz), Alkannawurzel, Laccainsäure (Stocklack), Indigo, Blauholzpulver, Krappwurzel- und Rhabarberwurzelpulver, etc.

Die erfindungsgemäßen Zusammensetzungen enthalten, insbesondere wenn es sich um Haartönungsmittel, die keine Shampoos sind, handelt, vorzugsweise zusätzlich noch mindestens ein kationisches Tensid, insbesondere in einer Menge von 0,1 bis 7,5, vorzugsweise 0,25 bis 5, besonders bevorzugt 0,5 bis 2,5 Gew.-%, der Gesamtzusammensetzung.

Geeignete langkettige quaternäre Ammoniumverbindungen, die als kationische Tenside allein oder im Gemisch miteinander eingesetzt werden können, sind insbesondere Cetyltrimethylammoniumchlorid, Dimethyldicetylammoniumchlorid, Trimethylcetylammoniumbromid, Stearyltrimethylammoniumchlorid, Dimethylstearylbenzylammoniumchlorid, Benzyltetradecyldimethylammoniumchlorid, Dimethyldihydriertes-Talgammoniumchlorid, Laurylpyridiniumchlorid, Lauryldimethylbenzylammoniumchlorid, Lauryltrimethylammoniumchlorid, Tris(oligooxy-. ethyl)alkylammoniumphosphat, Cetylpyridiniumchlorid, Behentrimoniumchlorid, etc. Gut geeignet sind auch die in der EP-A 472107 geoffenbarten quatemären Ammoniumsalze.
Im Prinzip sind alle quaternären Ammoniumverbindungen, wie sie im "CTFA International Cosmetic Ingredient Dictionary", Fourth Ed. (1991), unter dem Trivialnamen "Quaternium" aufgeführt sind, geeignet.

Die Zusammensetzung kann natürlich zusätzlich die in solchen Konditionierungsmitteln üblichen Bestandteile enthalten; es wird, zur Vermeidung von Wiederholungen, auf die Standard-Monographie K.Schrader, Grundlagen und Rezepturen der Kosmetika, 2. Aufl. (Hüthig Buch Verlag, 1989), S.722 - 771, verwiesen.

Auch nichtionische Tenside können, insbesondere im Gemisch mit kationaktiven Tensiden, Verwendung finden, beispielsweise Aminoxide in einer Menge von etwa 0,25 bis etwa 5, vorzugsweise etwa 0,5 bis etwa 3,5 Gew.-%, berechnet auf die Gesamtzusammensetzung des Mittels.

Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyl- oder -ethylaminoxide, C₁₂-C₁₈-Alkyldi(hydroxyethyl)- oder -(hydroxypropyl)aminoxide, oder auch Aminoxide mit Ethylenoxid- und /oder Propylenoxidgruppen in der Alkylkette.

Geeignete Tenside sind weiterhin die bekannten C₈-C₁₈-Alkylpolyglucoside, insbesondere mit einem Polykondensationsgrad von 1,2 bis 3.

Wenn es sich bei den erfindungsgemäßen Zusammensetzungen um Tönungsshampoos handelt, können auch anionische Tenside, vorzugsweise im Gemisch mit nichtionischen, amphoteren und/oder zwitterionischen Tensiden, eingesetzt werden.

Geeignete anionische Tenside sind natürlich diejenigen, die in Shampoos üblicherweise zum Einsatz gelangen, beispielsweise die bekannten C₁₀-C₁₈-Alkylsulfate und insbesondere die entsprechenden Ethersulfate, beispielsweise C₁₂-C₁₄-Alkylethersulfat, Laurylethersulfat, insbesondere mit 1 bis 4 Ethylenoxidgruppen im Molekül, weiterhin Monoglycerid(ether)sulfate, Fettsäureamidsulfate, die durch Ethoxylierung und anschließende Sulfatierung von Fettsäurealkanolamiden erhalten werden, und deren Alkalisalze sowie Salze langkettiger Mono- und Dialkylphosphate, die milde, hautverträgliche Detergentien darstellen.

Im Rahmen der Erfindung weiterhin geeignete anionische Tenside sind auch Isethionate, α-Olefinsulfonate bzw. deren Salze und insbesondere Alkalisalze von Sulfobernsteinsäurehalbestern, beispielsweise das Dinatriumsalz des Monooctylsulfosuccinats und Alkalisalze langkettiger Monoalkylethoxysulfosuccinate.

Geeignete Tenside vom Carboxylat-Typ sind Alkylpolyethercarbonsäuren und deren Salze der Formel

R-(C₂H₄O)ₙ-O-CH₂COOX,

worin R eine C₈-C₂₀-Alkylgruppe, vorzugsweise eine C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren der allgemeinen Formel 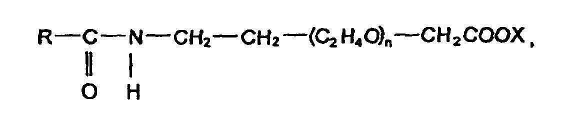 worin R und X die vorstehend angegebene Bedeutung haben und n insbesondere für eine Zahl von 1 bis 10, vorzugsweise 2,5 bis 5, steht.

Derartige Produkte sind seit längerem bekannt und im Handel, beispielsweise unter den Handelsnamen "AKYPO" und "AKYPO-SOFT®".

Es ist besonders zweckmäßig, Mischungen aus mehreren anionischen Tensiden einzusetzen, beispielsweise ein Gemisch aus einem α-Olefinsulfonat und einem Sulfosuccinat, vorzugsweise im Verhältnis von 1 : 3 bis 3 1, oder einem Ethersulfat und einer Polyethercarbonsäure oder Alkylamidoethercarbonsäure.

Im Gemisch mit anderen anionischen Tensiden ebenfalls einsetzbar sind Eiweiß-Fettsäure-Kondensationsprodukte an sich bekannter Struktur, insbesondere in Mengen zwischen etwa 0,5 und 5, vorzugsweise 1 bis 3 Gew.-% der Gesamtzusammensetzung des flüssigen Haarwaschmittels.

Eine Übersicht über die in flüssigen Körperreinigungsmitteln zum Einsatz gelangenden anionaktiven Tenside findet sich im übrigen in der Monographie von K.Schrader, S. 683 bis 691.

Der bevorzugte Mengenbereich an anionischen Tensiden in den erfindungsgemäßen flüssigen Körperreinigungsmitteln liegt zwischen etwa 2,5 und etwa 25 Gew.-%, insbesondere bei etwa 5 bis etwa 20 Gew.-%, besonders bevorzugt bei etwa 7,5 bis etwa 15 Gew.-%, berechnet auf die Gesamtzusammensetzung des Mittels.

Nichtionische Tenside gelangen in Tönungsshampoos vorzugsweise im Gemisch mit anionaktiven Tensiden zum Einsatz.

Ein bevorzugtes nichtionisches Tensid gehört dabei, wie bereits oben erwähnt, zu der Klasse der Alkylpolyglucoside der allgemeinen Formel

R-O-(R¹O)ₙ-Zₓ ,

worin R eine Alkylgruppe mit 8 bis 18 Kohlenstoffatomen, R¹ eine Ethylen- oder Propylengruppe, Z einen Saccharidrest mit 5 bis 6 Kohlenstoffatomen, n eine Zahl von 0 bis 10 und x eine Zahl zwischen 1 und 2,5 bedeuten.
Diese Alkylpolyglucoside sind in letzter Zeit insbesondere als ausgezeichnete hautverträgliche schaumverbessemde Mittel in flüssigen Wasch- und Körperreinigungsmitteln bekannt geworden und sind vorzugsweise in einer Menge von 1 bis 20, insbesondere 2,5 bis 15 Gew.-%, der Gesamtzusammensetzung enthalten.

Weitere nichtionische Tensidbestandteile in Tönungsshampoos sind beispielsweise langkettige Fettsäuremono- und -dialkanolamide, beispielsweise Cocosfettsäuremonoethanolamid und Myristinfettsäuremonoethanolamid, die auch als Schaumverstärker eingesetzt werden können.

Andere nichtionische Tenside sind beispielsweise die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics" im Verkehr sind.

Gemische aus anionaktiven Tensiden und Alkylpolyglucosiden, den bevorzugten nichtionischen Tensiden im Rahmen der Erfindung sowie deren Verwendung in flüssigen Körperreinigungsmitteln sind an sich bereits bekannt, beispielsweise aus der EP-A 70 074. Die dort beschriebenen Alkylpolyglucoside sind prinzipiell auch im Rahmen der vorliegenden Erfindung geeignet; ebenso die aus der EP-A 358 216 bekannten Gemische aus Sulfosuccinaten und Alkylpolyglucosiden.

Weitere im Gemisch mit anionaktiven Tensiden einsetzbare Tenside sind die bereits erwähnten Aminoxide in einer Menge von etwa 0,25 bis etwa 5, vorzugsweise etwa 0,5 bis etwa 3,5 Gew.-%, berechnet auf die Gesamtzusammensetzung des Mittels.

Als amphotere bzw. zwitterionische Tenside können beispielsweise Betaine, Sulfobetaine und/oder Alkylaminocarbonsäuren wie Alkylaminoglycinate, Alkylaminopropionate, Alkylglycinate, etc. eingesetzt werden. Besonders geeignet sind Alkylamidobetaine der allgemeinen Formel 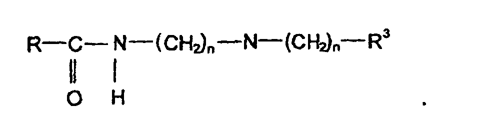 wobei R eine C₈-C₁₈-Alkylgruppe, z. B. einen Cocoalkylrest; R¹ und R² einen niederen C₁-C₄-Alkyl- oder -Hydroxyalkylrest, insbesondere eine Methyl-, Ethylund/oder Hydroxyethylgruppe; R³ eine COO⁻-oder-SO₃⁻-Gruppe; und n 1 bis 3 bedeuten.
Auch Betaine der allgemeinen Formel 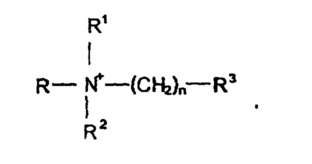 wobei R, R¹, R², R³ und n die obengenannte Bedeutung haben, sind bevorzugt.

Geeignet sind insbesondere auch Handelsprodukte wie beispielsweise "Tegobetaine®", "Dehytone®" wie "AB 30", "G" und "K", "Lonzaine®", "Varion®" wie "ADG" und "CAS", "Lexaine®", "Chembetaine®", "Mirataine®", "Rewoteric®", "Schercotaine®", "Monteine LCQ®", "Alkateric®", "Amonyl®", "Amphosol®", "Cycloteric BET®", "Emcol®", "Empigen®", "Mackam®", "Monateric®", "Unibetaine®" und "Velvetex®".

Die amphoteren bzw. zwitterionischen Tenside sind vorzugsweise in einer Menge von 0,25 bis 7,5 Gew.-%, insbesondere 0,5 bis 5 Gew.-%, berechnet auf die Gesamtzusammensetzung, enthalten.

Die erfindungsgemäßen Haarbehandlungsmittel können die in solchen wäßrigen Zubereitungen üblichen Stoffe enthalten.

Dies sind beispielsweise synthetische oder natürliche haarkonditionierende Polymere, vorzugsweise in einer Menge von 0,1 bis 2,5, insbesondere 0,25 bis 1,5 Gew.-% der Gesamtzusammensetzung.

Als geeignete kationische Polymere sind neben den altbekannten quatemären Cellulosederivaten des Typs "Polymer JR" insbesondere quatemisierte Homo- und Copolymere des Dimethyldiallylammoniumchlorids, wie sie unter dem Handelsnamen "Merquat" im Handel sind, quatemäre Vinylpyrrolidon-Copolymere, insbesondere mit Dialkylaminoalkyl(meth)acrylaten, wie sie unter dem Namen "Gafquat" bekannt sind, Copolymerisate aus Vinylpyrrolidon und Vinylimidazoliniummethochlorid, die unter dem Handelsnamen "Luviquat" angeboten werden, Polyamino-Polyamid-Derivate, beispielsweise Copolymere von Adipinsäure-Di methylaminohydroxypropyldiethylentriamin, wie sie unter dem Namen "Cartaretine F" vertrieben werden, sowie auch bisquatemäre langkettige Ammoniumverbindungen der in der US-PS 4157 388 beschriebenen Harnstoff-Struktur, die unter dem Handelsnamen "Mirapol A 15" im Handel sind, geeignet

Verwiesen wird in diesem Zusammenhang auch auf die in den DE-OSen 25 21 960, 2811 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden.

Anstelle der kationischen Polymeren oder in Kombination mit denselben können auch nichtionische Polymere verwendet werden. Als geeignete nichtionische Polymere werden vor allem Vinylpyrrolidon-Homo- und Copolymerisate, insbesondere Polyvinylpyrrolidon selbst, Copolymere aus Vinylpyrrolidon und Vinylacetat oder Terpolymerisate aus Vinylpyrrolidon, Vinylacetat und Vinylpropionat, wie sie beispielsweise von der Firma BASF unter dem Handelsnamen "Luviskol" vertrieben werden, eingesetzt.

Es können jedoch auch (Co-)Polymerisate aus den verschiedenen Acryl- und Methacrylestem, Acrylamid und Methacrylamid, beispielsweise Polyacrylamid mit Molgewichten von über 100.000, Dimethylhydantoin-Formaldehyd-Harze, etc., verwendet werden. Selbstverständlich sind auch Mischungen aus verschiedenen nichtionischen Polymeren verwendbar.

Geeignet sind schließlich auch noch amphotere Polymere, z. B. die unter der Bezeichnung "Amphomer" vertriebenen Copolymerisate aus N-Octylacrylamid, N-Butylaminoethylmethacrylat und Acrylsäure, sowie die bekannten anionischen Produkte, die im Stand der Technik hinreichend beschrieben sind.

Die erfindungsgemäßen Haarbehandlungsmittel können, wenn sie als Emulsionen, Dispersionen, Lösungen, Gele oder Aerosole als Nachbehandlungsmittel eingesetzt werden, die in solchen Zusammensetzungen üblichen Zusätze enthalten, deren Art und Charakter von der Applikationsform des Mittels abhängig ist. Es sind dies Fette, Fettalkohole, Emulgatoren, pH-Regulatoren, Lösungs- und Verdünnungsmittel, Lösungsvermittler, Konservierungsmittel, Parfums, etc.

Geeignete Fette und Öle, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.

Synthetische Öle und Wachse sind beispielsweise Silikonöle, Polyethylenglykole, etc.

Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc. Diese hydrophoben Komponenten sind in den erfindungsgemäßen Zusammensetzungen vorzugsweise in einer Gesamtmenge von etwa 0,5 bis etwa 10, insbesondere etwa 1 bis 7,5, vor allem etwa 1,5 bis 5 Gew.-%, berechnet auf die Gesamtzusammensetzung, enthalten.

Eine bevorzugte Gruppe von aktiven Ingredientien sind C₁₀-C₂₄-Fettsäuren, insbesondere Behensäure und Stearinsäure, vorzugsweise zwischen 0,1 und 10 Gew.-% der Gesamtzusammensetzung.

Ein weiterer bevorzugter Bestandteil ist ausgewählt aus der Gruppe Harnstoff, Benzylalkohol, Benzyloxyethanol, Phenylethylalkohol, Phenoxyethanol, 1-Methoxypropanol(-2), 1-Ethoxypropanol(-2), Diethylenglykolmonomethyl- oder ―ethylether, Dipropylenglykolmonomethyl- oder ―ethylether und/oder Zimtalkohol, insbesondere in einer Menge von 0,5 bis 15 Gew.-%, vorzugsweise etwa 1 bis 10 Gew.-% der Gesamtzusammensetzung.

Eine Zusammenfassung der Herstellung solcher Mittel findet sich in der bereits erwähnten Monographie von K. Schrader, S. 798 bis 815, insbesondere S. 804 ff.

Die erfindungsgemäßen Haarbehandlungmittel liegen als Emulsion, Dispersion oder (gegebenenfalls verdickte, d. h. als Gel) Lösung vor und können auch als Aerosolschaum konfektioniert werden. Diese Zusammensetzungen und ihre Herstellung sind dem Fachmann grundsätzlich bekannt und bedürfen daher keiner näheren Erläuterung.

Der pH-Wert der erfindungsgemäßen Haarfärbemittel liegt vorzugsweise bei 3 bis 8, insbesondere zwischen 4 und 6.

## Patentansprüche

1. Haarfärbemittel, enthaltend
a) mindestens einen Farbstoff, ausgewählt aus einer Verbindung der Gruppe 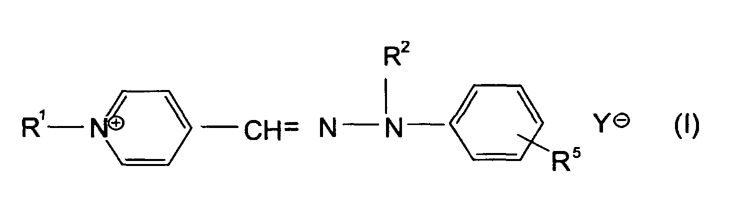 worin R¹ und R², für Wasserstoff, eine CH₃- oder C₂H₅-Gruppe stehen und R⁵ Wasserstoff -OCH3 oder -OC₂H₅ bedeutet,
b) mindestens einen anionischen UV-Absorber.

2. Haarfärbemittel nach Anspruch 1, enthaltend einen Farbstoff der allgemeinen Formel (I), worin R¹ und R² eine Methylgruppe, R⁵ Wasserstoff und Y⁻ eine Methosulfatgruppe bedeuten.

3. Haarfärbemittel nach Anspruch 1, enthaltend einen Farbstoff der allgemeinen Formel (I), worin R¹ und R² je eine Methylgruppe, R⁵ eine Methoxygruppe und Y⁻ Cl⁻ bedeuten.

4. Haarfärbemittel nach einem oder mehreren der Ansprüche 1 bis 3, enthaltend ein amphoteres und/oder zwitterionisches Tensid.

## Claims

1. Hair dyeing composition, comprising
a) at least one dyestuff selected from a compound of the group 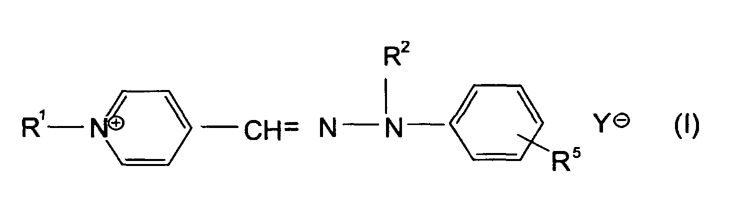 wherein R¹ and R² stand for hydrogen, a CH₃- or C₂H₅- group and
R⁵ stands for hydrogen -OCH3 or -OC₂H₅,
and
b) at least one anionic UV-absorber.

2. Hair dyeing composition according to claim 1, comprising a dyestuff of the general formula (I), wherein R¹ and R² stand for a methyl group, R⁵ stands for hydrogen, and Y⁻ stands for a methosulfate group.

3. Hair dyeing composition according to claim 1, comprising a dyestuff of the general formula (I), wherein R¹ and R² each stand for a methyl group, R⁵ stands for a methoxy group, and Y⁻ is Cl⁻.

4. Hair dyeing composition according to one or more of claims 1 to 3, comprising an amphoteric or zwitterionic surfactant.

## Revendications

1. Produit de coloration capillaire, contenant
a) au moins un colorant, choisi parmi un composé du groupe 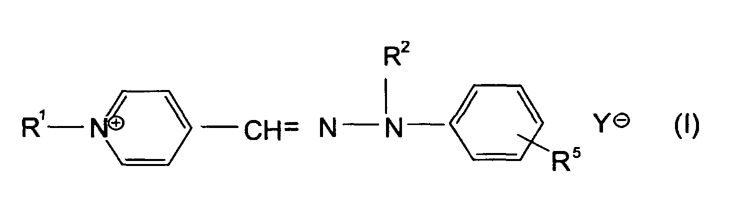 dans lequel R¹ et R² sont l'hydrogène, un groupe CH₃- ou un groupe C₂H₅- et R⁵ signifie l'hydrogène, -OCH3 ou -OC₂H₅,
et
b) au moins un absorbeur d'UV anionique.

2. Produit de coloration capillaire selon la revendication 1, contenant un colorant de formule générale (I), dans laquelle R¹ et R² signifient un groupe méthyle, R⁵ l'hydrogène et Y⁻ un groupe méthosulfate.

3. Produit de coloration capillaire selon la revendication 1, contenant un colorant de formule générale (I), dans laquelle R¹ et R² signifient un groupe méthyle, R⁵ un groupe méthoxy et Y⁻ signifie Cl⁻.

4. Produit de coloration capillaire selon l'une ou plusieurs des revendications 1 à 3, contenant un dérivé tensioactif amphotère et/ou zwitterionique.
